# EUROPEAN PATENT APPLICATION

(11) **EP 4 809 938 A1**
(43) Date of publication of application: **23.09.2026**
(21) Application number: 25217504.7
(22) Date of filing: 20.11.2025
(51) Int. Cl.: A61B 17/08, A61B 17/10, A61B 17/122, A61B 17/128

(54) **FLEXIBLE, ROTATABLE, AND REOPENABLE TRACTION-STYLE CONTINUOUS FIRING HEMOSTATIC CLIP**

(30) Priority: 17.03.2025 CN 202510312091; 27.08.2025 CN 202521828976 U; 15.09.2025 CN 202521983135 U
(71) Applicant: EASTERN ENTERPRISE CORPORATION, Beijing 100035 (CN)
(72) Inventor: SUN, Dong, Beijing, 100035 (CN); GUO, Jiansheng, Beijing, 100035 (CN); XING, Chao, Beijing, 100035 (CN)
(74) Representative: Bayramoglu et al.

(57) **Abstract**

Provided is a flexible, rotatable, and reopenable traction-style continuous firing hemostatic clip, which relates to the technical field of medical devices. The flexible, rotatable, and reopenable traction-style continuous firing hemostatic clip includes hemostatic clip assemblies sequentially connected within an outer tube assembly, and an inner guide assembly, where the hemostatic clip assemblies each include two hemostatic jaws, a hemostatic clip transmission member, and a hemostatic clip sleeve; the hemostatic jaws each include a front end provided with a buckle portion and a back end hinged to a front end of the hemostatic clip transmission member; a back portion of the hemostatic clip transmission member is provided with an engagement portion; the hemostatic clip transmission member passes through the hemostatic clip sleeve; the engagement portion is engaged with a buckle portion of an adjacent back hemostatic clip assembly; an opening/locking mechanism for opening/closing and locking the two hemostatic jaws is disposed between the two hemostatic jaws and the hemostatic clip sleeve; an outer side of the hemostatic clip sleeve is provided with a protruding portion; and a front end of the outer tube assembly is provided with a reduced-diameter portion adapted to the protruding portion.

## Description

### TECHNICAL FIELD

The present disclosure relates to the technical field of medical devices, and in particular to a flexible, rotatable, and reopenable traction-style continuous firing hemostatic clip. The present disclosure claims priority to: 1. Chinese Patent Application 2025103120910, titled "FLEXIBLE, ROTATABLE, AND REOPENABLE TRACTION-STYLE CONTINUOUS FIRING HEMOSTATIC CLIP", filed on March 17, 2025; 2. Chinese Patent Application 2025218289768, titled "FLEXIBLE, ROTATABLE, AND REOPENABLE ENDOSCOPIC CONTINUOUS FIRING TRACTION CLIP", filed on August 27, 2025; and 3. Chinese Patent Application 2025219831354, titled "FLEXIBLE, ROTATABLE, AND REOPENABLE ENDOSCOPIC CONTINUOUS FIRING FLUORESCENT MARKING CLIP", filed on September 15, 2025.

### BACKGROUND TECHNOLOGY

As a medical device, hemostatic clips are primarily used to: 1. provide endoscopic markers; 2. be utilized in procedures such as endoscopic submucosal dissection (ESD), endoscopic mucosal resection (EMR), and peroral endoscopic myotomy (POEM); 3. secure a jejunal feeding tube to a small intestinal wall; and 4. serve as an adjunctive treatment option for closing gastrointestinal luminal perforations of up to 20 mm under conservative management. Hemostatic clips are mainly employed in conjunction with endoscopes for bleeding control and are typically made of metal.

The current hemostatic clips on the market mainly come in two structural forms. 1) Detachable hemostatic clips: A single guidewire body is preloaded with one hemostatic clip and comes with multiple spare hemostatic clips. After one hemostatic clip is deployed into the human body, the guidewire is withdrawn from the endoscope. After a new hemostatic clip is loaded, the guidewire is reinserted into the endoscope. 2) Integrated hemostatic clips: A single guidewire body is preloaded with one hemostatic clip and is not equipped with, nor can it be reloaded with, other hemostatic clips. After the hemostatic clip is deployed into the human body, the guidewire is withdrawn from the endoscope and discarded. A new hemostatic clip is then used and inserted into the endoscope to repeat further procedures.

Current hemostatic clip designs on the market present the following issues. For detachable hemostatic clips, they are inconvenient to operate, and replacing hemostatic clips is cumbersome, which prolongs operation time, carries reloading risks, affects product functionality, and requires operator training. In addition, repeated insertions of the hemostatic clips into the endoscope during a single surgery can easily damage the endoscope and consume time and effort. For integrated hemostatic clips, each insertion allows deployment of only one hemostatic clip, and the guidewire is discarded after use, resulting in significant resource waste. In addition, the use of each new hemostatic clip requires repeating numerous steps, wasting valuable surgical time.

ESD procedures include two steps: circumferential incision and submucosal dissection, with the difficulty primarily determined by the submucosal dissection. In cases where tangential manipulation is challenging, poor visualization of the submucosal layer can easily lead to complications such as bleeding or perforation, which are difficult to manage.

To provide surgeons with adequate visual field for safe and effective dissection, traction clips are typically required. Traction clips are mainly used in ESD procedures for digestive tract lesions or when precise lesion localization is needed.

For example, the ZEOCLIP from Japan is used with S-O clips to achieve tissue traction through the following specific process. 1. The S-O clip is deployed. After circumferential incision around the lesion, a target position on the mucosal side of the lesion is selected to deploy the S-O clip. To avoid grasping the muscularis propria, a local injection can be first administered at the deployment site.

2. The ZEOCLIP is inserted and fixed. It is hooked onto the ring portion of the S-O clip, and then fixed to the digestive tract wall on the side opposite the lesion, slightly toward the oral side. During fixation, marking is performed first to confirm the direction and distance before proceeding, thereby ensuring effective traction.

3. Tissue dissection is performed. Under ZEOCLIP traction, the submucosal layer becomes directly visible, allowing the surgeon to perform dissection safely and easily.

4. Device retrieval is performed. After the lesion is resected, the ring portion of the S-O clip is cut, and retrieved together with the resected lesion tissue.

However, the existing traction clips have the following shortcomings.
1. Inconvenient package opening and operation. Taking the ZEOCLIP from Japan as an example, at least two traction clips are required, each individually packaged and requiring manual loading onto the clip applicator by medical staff, making the process cumbersome. The S-O clip is easily damaged during placement, with higher damage risk during stressful surgeries, potentially wasting valuable surgical time. After the first traction clip is loaded, it is inserted into the endoscope for clip application. This process is repeated to deploy the second traction clip, resulting in two insertions through the endoscopic instrument channel.
2. High operational difficulty requiring substantial training before proficiency.

During placement of the second traction clip, the traction clip must pass through the ring of the S-O clip and be pulled to the opposite side. This maneuver is difficult, requires operator training, and can only be performed effectively after proficiency is achieved.

In surgical procedures (open surgery, laparoscopic surgery), endoscopic devices (gastroscope, colonoscope) are often used to assist in confirming lesion locations. This involves placing a clip at the cancer site via the endoscope, with subsequent surgical confirmation of the clip's position through palpation. This proves challenging in surgeries that demand precise lesion localization, such as those for gastric cancer and digestive tract tumors.

### CONTENT OF THE INVENTION

The present disclosure provides a flexible, rotatable, and reopenable traction-style continuous firing hemostatic clip. The present disclosure achieves easy assembly of hemostatic clips and continuous deployment of multiple hemostatic clip assemblies. Therefore, the present disclosure simplifies cumbersome surgical steps for surgeons, reduces the operation time, minimizes resource waste, and enhances surgical efficiency, thereby lowering surgical costs. The present disclosure incorporates an elastic assembly between each two adjacent hemostatic clip assemblies, such that a front hemostatic clip assembly clamps a lesion tissue while a back hemostatic clip assembly clamps a normal tissue at an appropriate position on a digestive tract wall opposite the lesion tissue for traction, improving surgical efficiency. The hemostatic clip assembly with a fluorescent portion can accurately locate the tumor position, which reduces surgical risks and patient discomfort and improves the success rate of surgery.

The above technical objective of the present disclosure is achieved by the following technical solution.

A flexible, rotatable, and reopenable traction-style continuous firing hemostatic clip includes an outer tube assembly and multiple hemostatic clip assemblies disposed within the outer tube assembly, where the multiple hemostatic clip assemblies are connected sequentially end to end; the outer tube assembly is further provided therein with an inner guide assembly connected to a backmost hemostatic clip assembly; the hemostatic clip assemblies each include two symmetrically arranged hemostatic jaws, a hemostatic clip transmission member, and a hemostatic clip sleeve; the hemostatic jaws each include a front end provided with a buckle portion and a back end hinged to a front side of the hemostatic clip transmission member; a back side of the hemostatic clip transmission member is provided with an engagement portion; the hemostatic clip transmission member passes through the hemostatic clip sleeve, and the engagement portion extends beyond a back end of the hemostatic clip sleeve; the engagement portion is engaged with the buckle portion of an adjacent back hemostatic clip assembly; an opening/locking mechanism for opening/closing and locking the two hemostatic jaws is disposed between the two hemostatic jaws and the hemostatic clip sleeve; an outer side of the hemostatic clip sleeve is provided with a protruding portion; a front end of the outer tube assembly is provided with a reduced-diameter portion for limiting the hemostatic clip sleeve to enable opening/closing, locking, and rotation actions of the hemostatic clip assembly; and the reduced-diameter portion is adapted to the protruding portion.

In the aforementioned flexible, rotatable, and reopenable traction-style continuous firing hemostatic clip, the hemostatic jaws each include a hemostatic jaw plate and a hemostatic jaw tail plate fixedly connected to the hemostatic jaw plate; a front end of the hemostatic jaw plate is fixedly connected to a hemostatic jaw element; the buckle portion includes an engagement opening disposed in a middle portion of the hemostatic jaw element; the hemostatic jaw element is provided with clip teeth located on two sides of the engagement opening, respectively; and the clip teeth on the two symmetrically arranged hemostatic jaws mesh with each other.

In the aforementioned flexible, rotatable, and reopenable traction-style continuous firing hemostatic clip, the hemostatic clip transmission member includes a hemostatic clip transmission member body and a hinge portion fixedly connected to a front end of the hemostatic clip transmission member body; the engagement portion is an engagement groove disposed on a back end of the hemostatic clip transmission member body; the hemostatic jaw tail plate is provided with a through-hole close to an end side; and the two hemostatic jaw tail plates are rotatably connected to the hinge portion.

In the aforementioned flexible, rotatable, and reopenable traction-style continuous firing hemostatic clip, the hinge portion includes two hemostatic jaw connecting plates fixedly connected in parallel to the front end of the hemostatic clip transmission member body; the two hemostatic jaw tail plates are stacked between the two hemostatic jaw connecting plates; a first hinge shaft passes through the two hemostatic jaw connecting plates; and the first hinge shaft sequentially passes through the through-holes of the two hemostatic jaw tail plates.

In the aforementioned flexible, rotatable, and reopenable traction-style continuous firing hemostatic clip, the hinge portion includes a mounting element fixedly connected to the front end of the hemostatic clip transmission member body; two sides of the mounting element are vertically and fixedly connected to second hinge shafts, respectively; and the two second hinge shafts are embedded in the through-holes of the corresponding hemostatic jaw tail plates, respectively.

In the aforementioned flexible, rotatable, and reopenable traction-style continuous firing hemostatic clip, the hemostatic clip sleeve includes a hemostatic clip sleeve body; the protruding portion includes a first stop ring and a second stop ring disposed on a back end of the hemostatic clip sleeve body; the second stop ring is close to a back end of the hemostatic clip sleeve body; front sides of the first stop ring and the second stop ring are provided with inclined guide surfaces, respectively; and a front portion of the hemostatic clip sleeve body is axially provided with a pair of clearance grooves for the hemostatic jaw tail plates to pass through.

In the aforementioned flexible, rotatable, and reopenable traction-style continuous firing hemostatic clip, an outer diameter of the first stop ring is adapted to an inner diameter of the outer tube assembly; and the outer diameter of the first stop ring is not less than an outer diameter of the second stop ring.

In the aforementioned flexible, rotatable, and reopenable traction-style continuous firing hemostatic clip, the opening/locking mechanism includes opening/closing slots located in the hemostatic jaw tail plates, respectively; the opening/closing slots are located on front sides of the through-holes, respectively; the opening/closing slots are L-shaped; the opening/closing slots each include a locking segment and a free segment that transition smoothly; the locking segment is close to a side of the hemostatic jaw plate; the hemostatic clip transmission member is embedded in the hemostatic clip sleeve; a pin shaft passes through a front side of the hemostatic clip sleeve body; and the pin shaft sequentially passes through the opening/closing slots of the two hemostatic jaw tail plates.

In the aforementioned flexible, rotatable, and reopenable traction-style continuous firing hemostatic clip, the outer tube assembly includes an outer tube head and a first flexible tube; the reduced-diameter portion is located at a front end of the outer tube head; the reduced-diameter portion is a reduced-diameter conical ring extending forward; the reduced-diameter portion includes an outer conical surface and an inner conical surface; front ends of the outer conical surface and the inner conical surface form an annular front end surface; the reduced-diameter portion is circumferentially provided with multiple strip-shaped notches; the notches extend backward to the outer tube head; an inner side of a back portion of the outer tube head is provided with an inner boss; and a front end of the first flexible tube is provided with an outer step tightly fitted to the inner boss.

In the aforementioned flexible, rotatable, and reopenable traction-style continuous firing hemostatic clip, an inner side of a back end of the first flexible tube is tightly fitted and connected to a second flexible tube.

In the aforementioned flexible, rotatable, and reopenable traction-style continuous firing hemostatic clip, the inner guide assembly includes a coupling portion, a tube sleeve, and an inner guidewire; a front end of the coupling portion is engaged with the engagement groove of the backmost hemostatic clip assembly; the tube sleeve is connected to a back end of the coupling portion; and the inner guidewire is embedded and fixedly connected within the tube sleeve.

In the aforementioned flexible, rotatable, and reopenable traction-style continuous firing hemostatic clip, the coupling portion is an inner guide head; a front end of the inner guide head is provided with a guide groove engaged with the engagement groove of the backmost hemostatic clip assembly; a back end of the inner guide head is provided with a mounting hole; the tube sleeve is a flexible inner guide tube; the inner guidewire is embedded in the flexible inner guide tube; and a front end of the flexible inner guide tube and a front end of the inner guidewire are tightly fitted and embedded in the mounting hole.

In the aforementioned flexible, rotatable, and reopenable traction-style continuous firing hemostatic clip, the coupling portion includes a release clip and a sleeve ring; the release clip includes two symmetrically arranged release clip bodies; back portions of the two release clip bodies are fixedly connected to a clip back; front ends of the two release clip bodies are provided with opposite clip grooves; the clip grooves are engaged with the engagement groove of the backmost hemostatic clip assembly; middle portions of the two release clip bodies are provided with two opposite clamping grooves; the two clamping grooves are adapted to the sleeve ring; an outer diameter of the sleeve ring is adapted to an inner diameter of the outer tube assembly; the tube sleeve is a connecting tube; the clip back is fixedly connected to one end of the connecting tube; and another end of the connecting tube is fixedly connected to the inner guidewire.

In the aforementioned flexible, rotatable, and reopenable traction-style continuous firing hemostatic clip, an elastic assembly is hooked between two adjacent ones of the hemostatic clip assemblies; the elastic assembly includes a tension spring, an elastic ring, and a spring ring; one end of the elastic ring is fixedly connected to a connecting post; the connecting post is provided with a shaft hole; the connecting post is embedded in an inner cavity of the tension spring, and a spring shaft at one end of the tension spring is embedded in the shaft hole; an inner side of a middle portion of each of the hemostatic jaws is provided with a traction hole; a back end of the engagement portion is fixedly connected to a traction ring; a spring end ring at another end of the tension spring is hooked to the traction ring of a front hemostatic clip assembly; and the spring ring is hooked between the elastic ring and the traction hole of a back hemostatic clip assembly.

In the aforementioned flexible, rotatable, and reopenable traction-style continuous firing hemostatic clip, a front portion of each of the hemostatic jaws is provided with a fluorescent portion; the fluorescent portion is a strip-shaped piece made of a fluorescent material; the front portion of each of the hemostatic jaws is further provided with a through adhesive-filling hole; the fluorescent portion is attached to an outer wall of the hemostatic jaw and is fixedly connected to the hemostatic jaw through the adhesive-filling hole; and a front end of the fluorescent portion extends beyond the hemostatic jaw to form a pressing portion.

Overall, the present disclosure has the following beneficial technical effects.

In the present disclosure, the hemostatic clip assemblies are preloaded in the outer tube assembly in an end-to-end engagement manner. The inner guide assembly engages with the backmost hemostatic clip assembly. After the outer tube assembly is inserted into the endoscope, the preloaded hemostatic clip assemblies are sequentially released into the patient's body by pushing, pulling, and rotating the inner guide assembly. This simplifies cumbersome surgical steps for surgeons, reduces the operation time, minimizes resource waste, and enhances surgical efficiency, thereby lowering surgical costs.

In the present disclosure, the elastic assembly is disposed between each two adjacent hemostatic clip assemblies. After the front hemostatic clip assembly clamps the lesion tissue, the back hemostatic clip assembly clamps a normal tissue at an appropriate position on the digestive tract wall opposite the lesion tissue for traction. According to surgical needs, the above operations can be performed for multiple sets of hemostatic clip assemblies.

In the present disclosure, the hemostatic jaws with the fluorescent portions can accurately locate the tumor position, which makes the operation convenient and greatly shortens the operation time. The continuous firing feature greatly shortens the operation time for multiple insertions of hemostatic clip assemblies, avoiding the need for repeated passage through the endoscopic instrument channel, and thereby reducing surgical risks and patient discomfort and enabling precise, simple, and convenient operations.

### DESCRIPTION OF THE DRAWINGS

FIG. 1 is a structural view of Embodiment 1 of the present disclosure;
FIG. 2 is a structural view of Embodiment 1 of the present disclosure with an outer tube assembly removed;
FIG. 3 is a structural view of a hemostatic clip assembly in Embodiment 1 of the present disclosure;
FIG. 4 is an exploded view of the hemostatic clip assembly in Embodiment 1 of the present disclosure;
FIG. 5 is an exploded view of the outer tube assembly in Embodiment 1 of the present disclosure;
FIG. 6 is a structural view of an outer tube head in Embodiment 1 of the present disclosure;
FIG. 7 is a structural view of an inner guide assembly in Embodiment 1 of the present disclosure;
FIG. 8 is a cross-sectional view of the inner guide assembly in Embodiment 1 of the present disclosure;
FIG. 9 is an exploded view of the inner guide assembly in Embodiment 2 of the present disclosure;
FIG. 10 is an exploded view of the hemostatic clip assembly in Embodiment 2 of the present disclosure;
FIG. 11 is an exploded view of an elastic assembly in Embodiment 2 of the present disclosure;
FIG. 12 is a cross-sectional view of Embodiment 2 of the present disclosure;
FIG. 13 is a structural view of Embodiment 2 of the present disclosure with the outer tube assembly removed;
FIG. 14 is an exploded view of a hemostatic jaw and a fluorescent portion in Embodiment 3 of the present disclosure;
FIG. 15 is an exploded view of the hemostatic clip assembly in Embodiment 3 of the present disclosure;
FIG. 16 is a cross-sectional view of Embodiment 1 of the present disclosure with the hemostatic jaws closed;
FIG. 17 is a cross-sectional view of Embodiment 1 of the present disclosure when an inner conical surface of a reduced-diameter portion abuts against a guide surface of a first stop ring and the hemostatic jaws are opened;
FIG. 18 is a cross-sectional view of Embodiment 1 of the present disclosure when the first stop ring extends out of the outer tube head and the hemostatic jaws are opened;
FIG. 19 is a cross-sectional view of Embodiment 1 of the present disclosure when the hemostatic jaws are locked;
FIG. 20 is a cross-sectional view of Embodiment 1 of the present disclosure when a locked hemostatic clip assembly is released by a subsequent hemostatic clip assembly;
FIG. 21 is a cross-sectional view of a backmost hemostatic clip assembly in Embodiment 1 of the present disclosure after the locked hemostatic clip assembly is released;
FIG. 22 is a cross-sectional view of Embodiment 1 of the present disclosure when the backmost hemostatic clip assembly is retracted into the outer tube assembly;
FIG. 23 is a structural view of Embodiment 2 of the present disclosure when the hemostatic jaws of the hemostatic clip assembly extend out of the outer tube head and are opened;
FIG. 24 is a structural view of Embodiment 2 of the present disclosure when the first stop ring of the hemostatic clip sleeve extends out of the outer tube head;
FIG. 25 is a structural view of Embodiment 2 of the present disclosure when the hemostatic jaws of the hemostatic clip assembly are locked;
FIG. 26 is a structural view of Embodiment 2 of the present disclosure when the hemostatic jaws of the back hemostatic clip assembly extend out of the outer tube head and release the front hemostatic clip assembly;
FIG. 27 is a structural view of Embodiment 2 of the present disclosure when the first stop ring of the hemostatic clip sleeve in the back hemostatic clip assembly extends out of the outer tube head;
FIG. 28 is a structural view of Embodiment 2 of the present disclosure during traction and lifting;
FIG. 29 is a structural view of Embodiment 2 of the present disclosure after traction and lifting and locking the hemostatic jaws of the back hemostatic clip assembly;
FIG. 30 is a structural view of Embodiment 2 of the present disclosure when a release clip extends out of the outer tube head and releases the back hemostatic clip assembly; and
FIG. 31 is a structural view of Embodiment 2 of the present disclosure when an elastic ring of the elastic assembly is cut.

Reference Numerals: 1. outer tube assembly; 11. outer tube head; 111. reduced-diameter portion; 1111. outer conical surface; 1112. inner conical surface; 1113. front end surface; 112. notch; 113. inner boss; 12. first flexible tube; 121. outer step; 13. second flexible tube; 14. soft rubber coating; 2. hemostatic clip assembly; 21. hemostatic jaw; 211. hemostatic jaw plate; 2111. adhesive-filling hole; 2112. limit protrusion; 212. hemostatic jaw tail plate; 2121. through-hole; 2122. traction hole; 213. hemostatic jaw element; 2131. engagement opening; 2132. clip tooth; 22. hemostatic clip transmission member; 221. hemostatic clip transmission member body; 2211. engagement groove; 2212. traction ring; 222. hinge portion; 2221. hemostatic jaw connecting plate; 2222. first hinge shaft; 2223. hinge shaft hole; 2224. mounting element; 2225. second hinge shaft; 23. hemostatic clip sleeve; 231. hemostatic clip sleeve body; 2311. first stop ring; 2312. second stop ring; 2313. clearance groove; 2314. pin shaft hole; 3. inner guide assembly; 31. coupling portion; 311. inner guide head; 3111. guide groove; 3112. mounting hole; 312. release clip; 3121. release clip body; 3122. clip groove; 3123. clamping groove; 3124. clip back; 313. sleeve ring; 32. tube sleeve; 321. flexible inner guide tube; 322. connecting tube; 33. inner guidewire; 4. opening/locking mechanism; 41. opening/closing slot; 411. locking segment; 412. free segment; 42. pin shaft; 5. elastic assembly; 51. tension spring; 511. spring shaft; 512. spring end ring; 52. elastic ring; 521. connecting post; 5211. shaft hole; 53. spring ring; 6. fluorescent portion; and 61. pressing portion.

### SPECIFIC IMPLEMENTATIONS

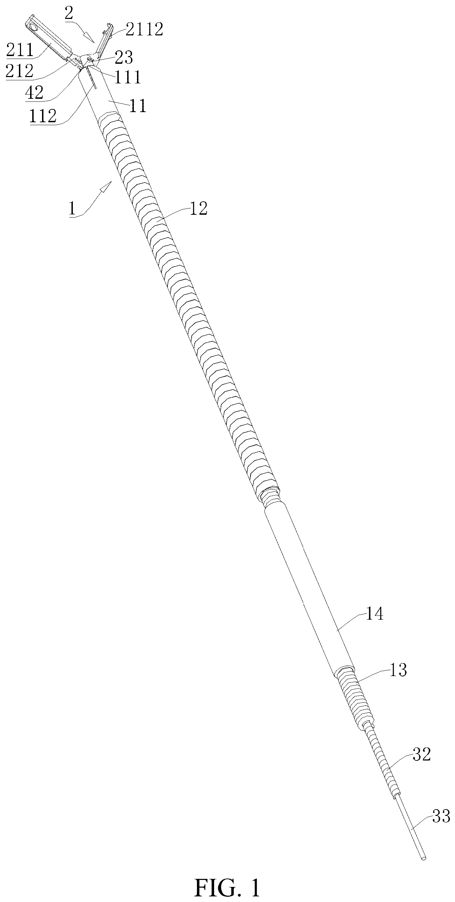

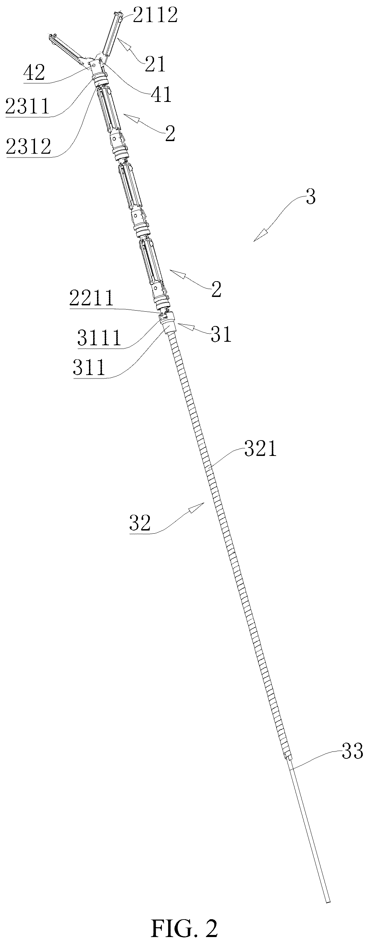

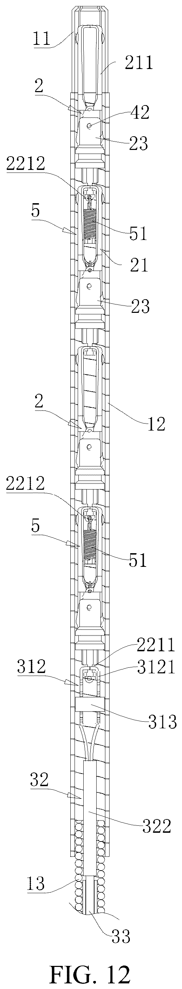

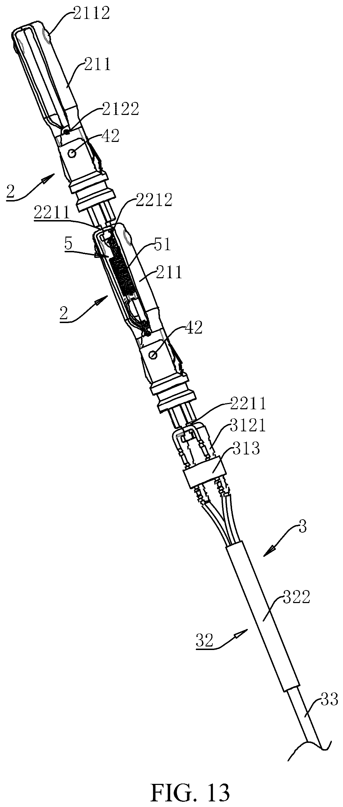

The present disclosure will be further described in detail below with reference to FIGS. 1 to 31.

### Embodiment 1

As shown in FIG. 1 to FIG. 8, a flexible, rotatable, and reopenable traction-style continuous firing hemostatic clip includes outer tube assembly 1 and multiple hemostatic clip assemblies 2 disposed within the outer tube assembly 1. The multiple hemostatic clip assemblies 2 are connected sequentially end to end. The outer tube assembly 1 is further provided therein with inner guide assembly 3 connected to the backmost hemostatic clip assembly 2.

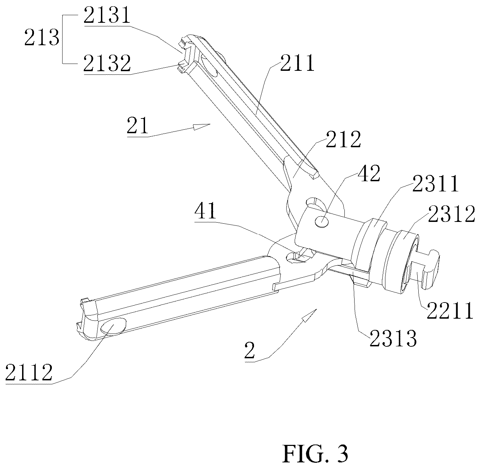

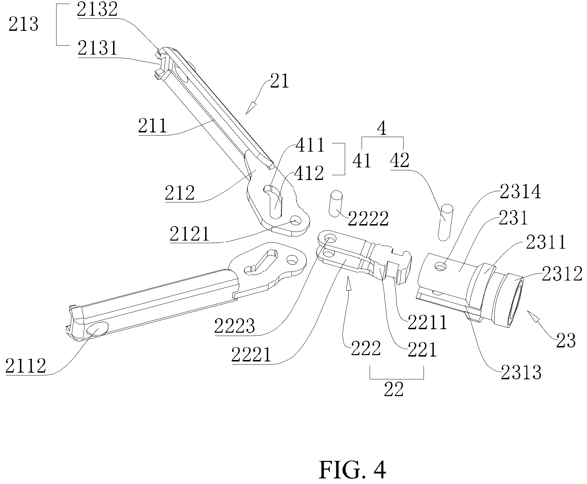

As shown in FIG. 3 and 4, the hemostatic clip assemblies 2 each include two symmetrically arranged hemostatic jaws 21, hemostatic clip transmission member 22, and hemostatic clip sleeve 23. The two hemostatic jaws 21 each include a front end provided with a buckle portion and a back end hinged to a front side of the hemostatic clip transmission member 22. A back side of the hemostatic clip transmission member 22 is provided with an engagement portion. The hemostatic clip transmission member 22 passes through the hemostatic clip sleeve 23 and the engagement portion extends beyond a back end of the hemostatic clip sleeve 23. The engagement portion is engaged with the buckle portion of the adjacent back hemostatic clip assembly 2. Opening/locking mechanism 4 for opening/closing and locking the two hemostatic jaws 21 is disposed between the two hemostatic jaws 21 and the hemostatic clip sleeve 23. An outer side of the hemostatic clip sleeve 23 is provided with a protruding portion. A front end of the outer tube assembly 1 is provided with reduced-diameter portion 111 for limiting the hemostatic clip sleeve 23 to enable opening/closing, locking, and rotation actions of the hemostatic clip assembly 2. The reduced-diameter portion 111 is adapted to the protruding portion.

The hemostatic jaws 21 each include hemostatic jaw plate 211 and hemostatic jaw tail plate 212 fixedly connected to the hemostatic jaw plate. A front end of the hemostatic jaw plate 211 is fixedly connected to hemostatic jaw element 213. The buckle portion includes engagement opening 2131 disposed in a middle portion of the hemostatic jaw element 213. The engagement opening 2131 is U-shaped. The hemostatic jaw element 213 is provided with clip teeth 2132 located on two sides of the engagement opening 2131, respectively. The clip teeth 2132 on the two symmetrically arranged hemostatic jaws 21 mesh with each other. The mutually engaged clip teeth 2132 are configured to clamp a tissue and prevent the hemostatic clip assembly 2 from falling off.

A thickness of the hemostatic jaw element 213 is less than a width of engagement groove 2211, such that a gap is formed between the engagement opening 2131 of the hemostatic jaw plate 211 and the engagement groove 2211. This ensures that two adjacent ones of the hemostatic clip assemblies 2 can bend along with the bending of the outer tube assembly 1.

The hemostatic clip transmission member 22 includes hemostatic clip transmission member body 221 and hinge portion 222 fixedly connected to a front end of the hemostatic clip transmission member body. The engagement portion is the engagement groove 2211 disposed on a back end of the hemostatic clip transmission member body 221. The hemostatic jaw tail plate 212 is provided with through-hole 2121 close to an end side. The two hemostatic jaw tail plates 212 are rotatably connected to the hinge portion 222.

In Embodiment 1, as shown in FIG. 4, the hinge portion 222 includes two hemostatic jaw connecting plates 2221 fixedly connected in parallel to the front end of the hemostatic clip transmission member body 221. The two hemostatic jaw tail plates 212 are stacked between the two hemostatic jaw connecting plates 2221. First hinge shaft 2222 passes through the two hemostatic jaw connecting plates 2221. Specifically, the first hinge shaft 2222 sequentially passes through through-holes 2121 of the two hemostatic jaw tail plates 212. The two hemostatic jaws 21 are rotatable around the first hinge shaft 2222 to enable the opening and closing actions of the two hemostatic jaws 21.

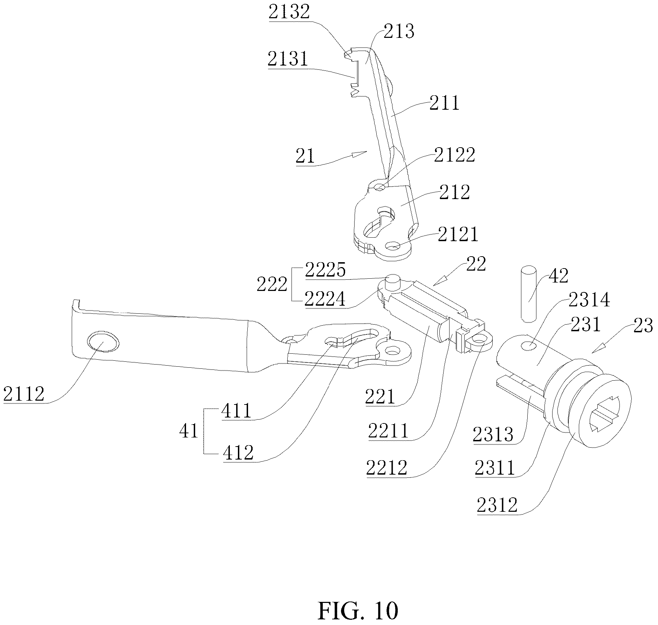

In Embodiments 2 and 3, as shown in FIG. 10, the hinge portion 222 includes mounting element 2224 fixedly connected to the front end of the hemostatic clip transmission member body 221. Two sides of the mounting element 2224 are vertically and fixedly connected to second hinge shafts 2225, respectively. The two second hinge shafts 2225 are embedded in the through-holes 2121 of the corresponding hemostatic jaw tail plates 212, respectively. The two hemostatic jaws 21 are respectively rotatable around the second hinge shafts 2225 to enable the opening and closing actions of the two hemostatic jaws 21.

When the two symmetrically arranged hemostatic jaws 21 are closed, the two engagement openings 2131 form a rectangular opening. The engagement groove 2211 is provided with a square cross-section. The two engagement openings 2131 engage with the engagement groove 2211 of the hemostatic clip transmission member 22 in the front hemostatic clip assembly 2, forming the multiple hemostatic clip assemblies 2 connected sequentially end to end by snap-fitting.

The hemostatic clip sleeve 23 includes cylindrical hemostatic clip sleeve body 231. The protruding portion includes first stop ring 2311 and second stop ring 2312 disposed on a back end of the hemostatic clip sleeve body 231. The second stop ring 2312 is close to the back end of the hemostatic clip sleeve body 231. Front sides of the first stop ring 2311 and the second stop ring 2312 are provided with inclined guide surfaces, respectively. A front portion of the hemostatic clip sleeve body 231 is axially provided with a pair of clearance grooves 2313 for the hemostatic jaw tail plates 212 to pass through.

An outer diameter of the first stop ring 2311 is adapted to an inner diameter of the outer tube assembly 1. The outer diameter of the first stop ring 2311 is not less than an outer diameter of the second stop ring 2312.

A thrust required to push the first stop ring 2311 out of the outer tube head 11 is denoted as F1, and a thrust required to push the second stop ring 2312 out of the outer tube head 11 is denoted as F2. A thrust required to unlock the two hemostatic jaws 21 from a freely locked state is denoted as F3, and a thrust required to unlock the two hemostatic jaws 21 from a locked state of clamping the tissue is denoted as F4. These four thrusts satisfy: F4 > F1 ≥ F2 > F3.

The opening/locking mechanism 4 includes opening/closing slots 41 disposed on the hemostatic jaw tail plates 212, respectively. The opening/closing slots 41 are located on front sides of the through-holes 2121, respectively. The opening/closing slots 41 are L-shaped. The opening/closing slots 41 each include locking segment 411 and free segment 412 that transition smoothly. The locking segment 411 is close to a side of the hemostatic jaw plate 211. The hemostatic clip transmission member 22 is embedded in the hemostatic clip sleeve 23. The hemostatic jaw tail plates 212 of the two hemostatic jaws 21 are embedded in the corresponding clearance grooves 2313, respectively. Pin shaft 42 passes through a front side of the hemostatic clip sleeve body 231. The pin shaft 42 sequentially passes through the opening/closing slots 41 of the two hemostatic jaw tail plates 212.

During specific mounting, the two hemostatic jaws 21 are opened. The pin shaft 42 is easily passed through the free segments 412 of the opening/closing slots 41 of the two hemostatic jaw tail plates 212. Then the two hemostatic jaws 21 are locked, such that the pin shaft 42 is located in the locking segments 411 of the opening/closing slots 41 and placed into the outer tube assembly 1.

The engagement openings 2131 of the two hemostatic jaw plates 211 engage with the engagement groove 2211 of the front hemostatic clip assembly 2. This allows the transmission of the thrust, a pulling force, and a rotational force forward, enabling the opening/closing, locking, forward/backward movement, and rotation of the hemostatic jaw plates 211.

When the hemostatic clip assemblies 2 are assembled, the engagement groove 2211 of the front hemostatic clip assembly 2 is engaged with the engagement openings 2131 of the adjacent back hemostatic jaws 21. The two engagement openings 2131 form a closed rectangular opening when the hemostatic jaws 21 are closed. The two adjacent hemostatic clip assemblies 2 are not detached when the hemostatic jaws 21 are closed.

When the inner guide assembly 3 is pushed and pulled back and forth, repeated opening and closing of the two hemostatic jaws 21 can be achieved if the pin shaft 42 moves within the free segments 412 of the opening/closing slots 41.

If the pin shaft 42 moves to the locking segments 411 of the opening/closing slots 41, the two hemostatic jaws 21 are in a locked state.

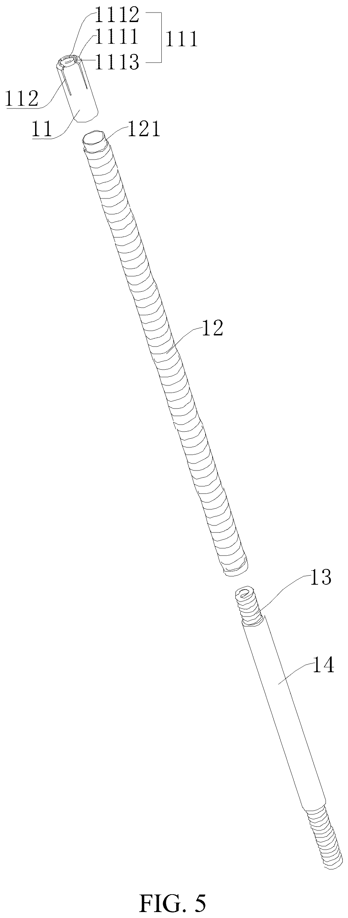

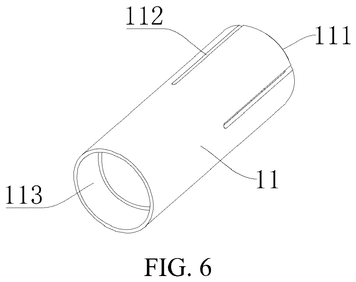

As shown in FIG. 5 and FIG. 6, the outer tube assembly 1 includes outer tube head 11 and first flexible tube 12. The reduced-diameter portion 111 is located at a front end of the outer tube head 11. The reduced-diameter portion 111 is a reduced-diameter conical ring extending forward. The reduced-diameter portion 111 includes outer conical surface 1111 and inner conical surface 1112. Front ends of the outer conical surface 1111 and the inner conical surface 1112 form annular front end surface 1113. The reduced-diameter portion 111 is circumferentially provided with multiple strip-shaped notches 112. The notches 112 extend backward to the outer tube head 11. An inner side of a back portion of the outer tube head 11 is provided with inner boss 113. A front end of the first flexible tube 12 is provided with outer step 121 tightly fitted to the inner boss 113. After the outer tube head 11 and the first flexible tube 12 are tightly fitted, their connection robustness can be improved by a fixed connection method. An inner diameter of the outer tube head 11 is equal to an inner diameter of the first flexible tube 12, ensuring that the hemostatic clip assemblies 2 can move stably and smoothly within an inner cavity of the outer tube assembly 1.

Through the above design, when the hemostatic jaws 21 clamp a tissue and is locked, if the inner guide assembly 3 is pushed forward, the thrust required to push the second stop ring 2312 of the protruding portion out of the outer tube head 11 is less than the thrust required to unlock the hemostatic jaws 21 from the locked state of clamping the tissue. The design allows the release of the locked hemostatic jaws 21.

The outer tube head 11 and the reduced-diameter portion 111 are integrally formed and made of medical stainless steel. The outer tube head 11 is cylindrical. The conical surface structure of the reduced-diameter portion 111 facilitates its entry into an endoscope and serves as a control structure to offer cooperation in achieving various actions of the hemostatic clip assembly 2.

An inner side of a back end of the first flexible tube 12 is tightly fitted and connected to a second flexible tube 13.

The first flexible tube 12 is a spiral flat-wire spring tube. Its inner wall is relatively flat and includes multiple gaps, enabling bending at a certain angle. Thus, the hemostatic clip assembly 2 can move back and forth easily inside the first flexible tube. The first flexible tube 12 can deform according to the shape of an object and return to its original state after passing through the object. The first flexible tube 12 can also be made of other flexible materials. The second flexible tube 13 is a spiral round-wire spring tube. Considering that the outer surface of the round-wire spring tube has a continuous undulating profile, soft rubber coating 14 is applied to an outer side of the round-wire spring tube. The soft rubber coating 14 effectively increases the tensile resistance of the second flexible tube and reduces its friction coefficient, thereby reducing friction damage caused to an inner wall of an endoscopic instrument channel.

The inner guide assembly 3 includes coupling portion 31, tube sleeve 32, and inner guidewire 33. A front end of the coupling portion 31 is engaged with the engagement groove 2211 of the backmost hemostatic clip assembly 2. The tube sleeve 32 is connected to a back end of the coupling portion 31. The inner guidewire 33 is embedded and fixedly connected within the tube sleeve 32.

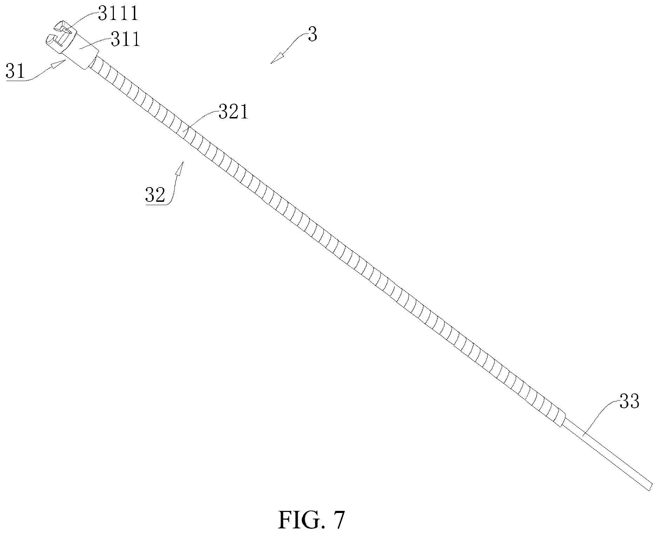

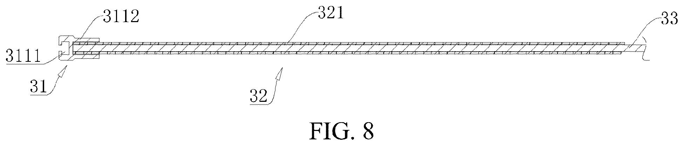

As shown in FIG. 7 and FIG. 8, in Embodiment 1, the coupling portion 31 is inner guide head 311. A front end of the inner guide head 311 is provided with guide groove 3111 engaged with the engagement groove 2211 of the backmost hemostatic clip assembly 2. A back end of the inner guide head 311 is provided with mounting hole 3112. The tube sleeve 32 is flexible inner guide tube 321. The inner guidewire 33 is embedded in the flexible inner guide tube 321. A front end of the flexible inner guide tube 321 and a front end of the inner guidewire 33 are tightly fitted and embedded in the mounting hole 3112.

An outer diameter of the flexible inner guide tube 321 is adapted to an inner diameter of the second flexible tube 13, ensuring stable and smooth pushing of the inner guide assembly 3 within the second flexible tube 13.

The flexible inner guide tube 321 improves the structural strength of the inner guide assembly 3 without affecting its bending ability. It can prevent the inner guide assembly 3 from bending due to a thrust within the first flexible tube 12.

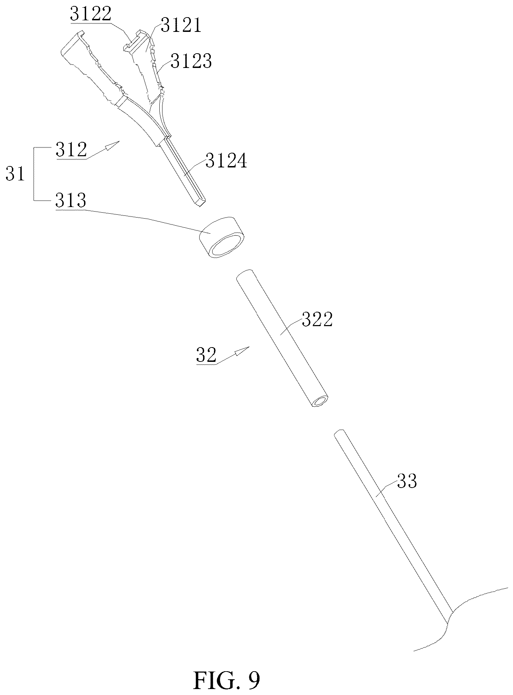

As shown in FIG. 9, in Embodiments 2 and 3, the coupling portion 31 includes release clip 312 and sleeve ring 313. The release clip 312 includes two symmetrically arranged release clip bodies 3121. Back portions of the two release clip bodies 3121 are fixedly connected to clip back 3124. Front ends of the two release clip bodies 3121 are provided with two opposing clip grooves 3122, respectively. The two clip grooves 3122 are engaged with the engagement groove 2211 of the backmost hemostatic clip assembly 2. Middle portions of the two release clip bodies 3121 are provided with opposing clamping grooves 3123, respectively. The clamping grooves 3123 are adapted to the sleeve ring 313. An outer diameter of the sleeve ring 313 is adapted to an inner diameter of the outer tube assembly 1. The tube sleeve 32 is connecting tube 322. The clip back 3124 is fixedly connected to one end of the connecting tube 322. Another end of the connecting tube 322 is fixedly connected to the inner guidewire 33.

The sleeve ring 313 is sleeved on the clamping grooves 3123 of the release clip body 3121 to keep the release clip 312 in a closed state.

A thickness of the clip groove 3122 at the front end of the release clip body 3121 is less than a thickness of the engagement groove 2211. A gap is formed between the clip groove 3122 and the engagement groove 2211 of the hemostatic clip transmission member 22 in the backmost hemostatic clip assembly 2, facilitating bending of the backmost hemostatic clip assembly 2 and the release clip 312.

The reduced-diameter portion 111 and the outer tube head 11 are provided with the multiple notches 112. When the inner guide assembly 3 is pushed forward, the hemostatic clip assembly 2 moves forward. The inner conical surface 1112 of the reduced-diameter portion 111 contacts the guide surface on the front side of the first stop ring 2311 on the outer tube head 11, causing the reduced-diameter portion 111 and the outer tube head 11 to expand outward, thereby pushing the first stop ring 2311 out of the reduced-diameter portion 111. The reduced-diameter portion 111 is caught between the first stop ring 2311 and the second stop ring 2312. At this time, by pushing, pulling, and rotating the inner guide assembly 3, the hemostatic jaws 21 are opened, closed, and rotated. When the hemostatic jaw 21 needs to clamp a tissue, the inner guide assembly 3 is pulled backward. The hemostatic jaws 21 receive a large pulling force from the hemostatic clip transmission member 22, and thus the two hemostatic jaws 21 are pulled backward. The pin shaft 42 moves from the free segments 412 of the opening/closing slots 41 to the locking segments 411 of the opening/closing slots 41, thereby locking the hemostatic jaw 21. Then the inner guide assembly 3 is pushed forward. The guide surface on the front side of the second stop ring 2312 cooperates with the inner conical surface 1112 of the reduced-diameter portion 111 until the second stop ring 2312 expands the outer tube head 11 outward. Thus, the second stop ring 2312 is pushed out of the reduced-diameter portion 111, finally causing the hemostatic clip assembly 2 to fully extend out of the outer tube head 11. The inner guide assembly 3 is continuously pushed, and the back hemostatic jaws 21 extend out of the outer tube head 11 and are opened, thereby fully releasing the front hemostatic clip assembly 2.

Driven by the inner guide assembly 3, the hemostatic clip assemblies 2 are sequentially extended out of the outer tube head 11 of the outer tube assembly 1. The hemostatic clip assembly 2 extending out of the outer tube head 11 performs a 360° rotational movement along the front end of the outer tube head 11 under the action of the inner guide assembly 3, thereby opening or closing the hemostatic jaws 21.

When the reduced-diameter portion 111 is located between the first stop ring 2311 and the second stop ring 2312 of the hemostatic clip sleeve body 231, moving back and forth or rotating the inner guide assembly 3 allows repeated opening/closing, locking, and free rotation of the hemostatic jaws 21.

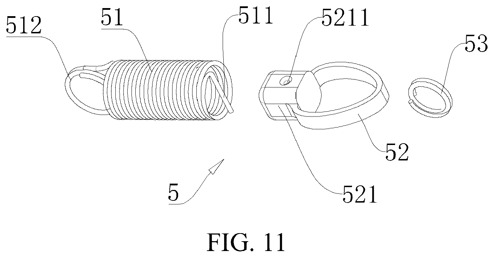

As shown in FIG. 11 to FIG. 13, in Embodiment 2, elastic assembly 5 is hooked between two adjacent ones of the hemostatic clip assemblies 2. The elastic assembly 5 includes tension spring 51, elastic ring 52, and spring ring 53. One end of the elastic ring 52 is fixedly connected to connecting post 521. The connecting post 521 is provided with shaft hole 5211. The connecting post 521 is embedded in an inner cavity of the tension spring 51, and spring shaft 511 at one end of the tension spring 51 is embedded in the shaft hole 5211. An inner side of a middle portion of the hemostatic jaw 21 is provided with traction hole 2122. A back end of the engagement portion is fixedly connected to the traction ring 2212. Spring end ring 512 at another end of the tension spring 51 is hooked to the traction rings 2212 of the front hemostatic clip assembly 2. The spring ring 53 is hooked between the elastic ring 52 and the traction hole 2122 of the back hemostatic clip assembly 2.

The tension spring 51 and the elastic ring 52 of the elastic assembly 5 provide elasticity, and they can be non-spring elastic members, such as silicone wires, to provide elasticity, or can be non-elastic members, such as medical sutures.

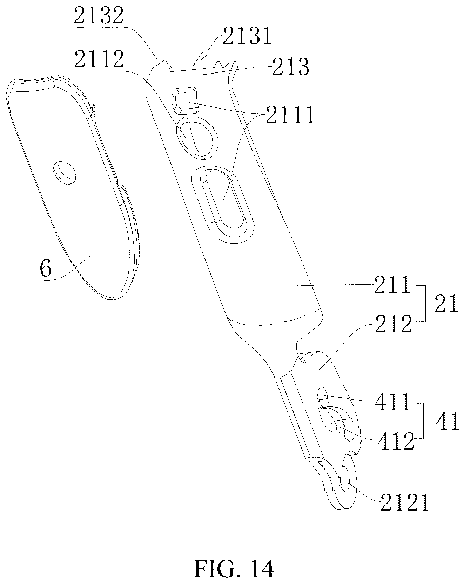

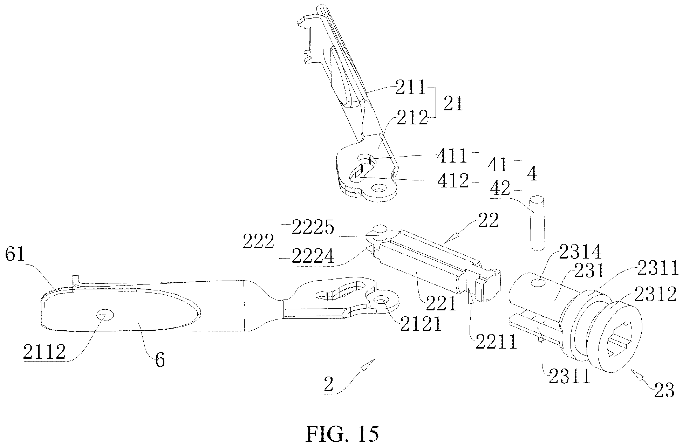

As shown in FIG. 14 and FIG. 15, in Embodiment 3, a front portion of the hemostatic jaw 21 is provided with fluorescent portion 6. The fluorescent portion 6 is a strip-shaped piece made of a fluorescent material. The front portion of the hemostatic jaw 21 is further provided with through adhesive-filling hole 2111. The fluorescent portion 6 is attached to an outer wall of the hemostatic jaw 21 and is fixedly connected to the hemostatic jaw 21 through the adhesive-filling hole 2111. A front end of the fluorescent portion 6 extends beyond the hemostatic jaw 21 to form pressing portion 61.

In the hemostatic clip assembly 2 with the fluorescent portion 6, after the clip teeth 2132 clamp the edge of a lesion tissue, the pressing portion 61 of the fluorescent portion 6 can press the digestive tract mucosa at the edge of the lesion tissue to make it thinner. This makes it easier for the fluorescent portion 6 to receive excitation light and emit fluorescence, thereby accurately locating the tumor position.

A front portion of the hemostatic jaw plate 211 is provided with limit protrusion 2112. The limit protrusion 2112 passes through the fluorescent portion 6 and protrudes from the fluorescent portion 6. The limit protrusion 2112 abuts against an inner wall of the outer tube assembly 1. The limit protrusion 2112 is constrained by the inner wall of the outer tube assembly 1, which prevents the engagement opening 2131 on the hemostatic jaw element 213 from disengaging from the engagement groove 2211. This ensures that the thrust, pulling force, and torque of the inner guide assembly 3 can be transmitted sequentially to the front hemostatic clip assembly 2.

In Embodiment 1, an assembly process of the hemostatic clip assembly 2 is as follows. First, the two hemostatic jaw tail plates 212 are stacked between the two hemostatic jaw connecting plates 2221. The two hemostatic jaw connecting plates 2221 are provided with coaxial hinge shaft holes 2223. The through-holes 2121 of the two hemostatic jaw tail plates 212 are aligned with the hinge shaft holes 2223 of the two hemostatic jaw connecting plates 2221. The first hinge shaft 2222 is passed through the two hinge shaft holes 2223 and fastened to the hemostatic jaw connecting plates 2221. Then the hemostatic clip transmission member 22 is inserted from the front end of the hemostatic clip sleeve 23, and the hemostatic jaw tail plates 212 are placed in the clearance grooves 2313. The hemostatic clip sleeve body 231 is provided with pin shaft hole 2314. The pin shaft hole 2314 is aligned with the two opening/closing slots 41. Preferably, the pin shaft hole is aligned with the free segments 412 of the opening/closing slots 41. The pin shaft 42 is passed through the pin shaft hole 2314 and is fixed.

All the hemostatic jaws 21 in the outer tube assembly 1 are in a closed but non-locked state. The hemostatic jaws 21 are closed but are not elastically deformed. No matter how long they are stored, the opening angle of the hemostatic jaws 21 will not be affected during use.

In Embodiment 1, when the hemostatic clip is in use, the hemostatic clip is first inserted into a human body through an endoscope and is placed nearby a mucosal tissue or blood vessel that requires hemostasis. The hemostatic clip assembly 2 is slowly pushed out from the outer tube head 11, and the reduced-diameter portion 111 is located between the first stop ring 2311 and the second stop ring 2312 of the protruding portion. At this time, the hemostatic jaws 21 are in an open state. When the clamping position of the hemostatic jaws 21 on the tissue wound or blood vessel is not ideal, the inner guide assembly 3 is pushed, pulled, and rotated to adjust the hemostatic jaws 21 to an ideal clamping position and then lock the hemostatic jaws, thereby ensuring the hemostatic effect.

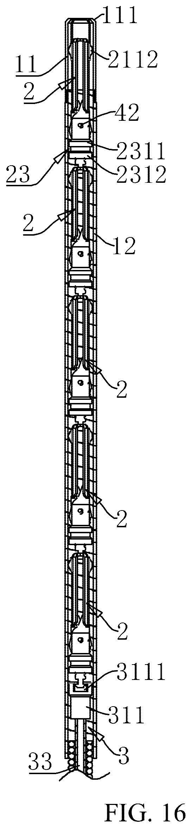

As shown in FIG. 16-22, a specific usage method of Embodiment 1 is as follows.

As shown in FIG. 16, the hemostatic clip assembly 2 and the inner guide assembly 3 are located within the outer tube assembly 1.

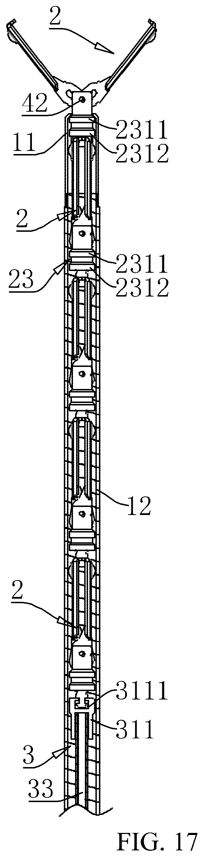

As shown in FIG. 17, in step 1, the inner guide assembly 3 is pushed forward to slowly push the frontmost hemostatic clip assembly 2 out of the outer tube head 11. When the inner conical surface 1112 of the reduced-diameter portion 111 blocks the guide surface of the first stop ring 2311, the two hemostatic jaws 21 are opened.

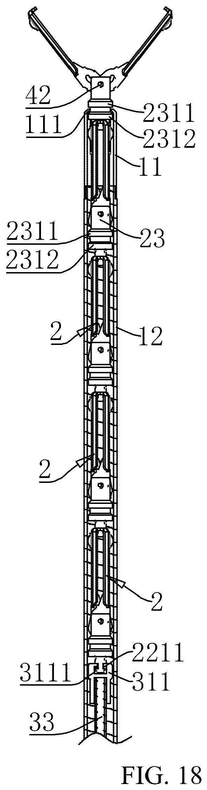

As shown in FIG. 18, in step 2, due to the multiple notches 112 of the reduced-diameter portion 111 and the outer tube head 11, the front ends of the reduced-diameter portion 111 and the outer tube head 11 are expanded outward. The inner guide assembly 3 is continuously pushed forward to push the first stop ring 2311 of the protruding portion to the front side of the front end surface 1113 of the reduced-diameter portion 111. At this time, the inner guide assembly 3 can be freely rotated to drive the frontmost hemostatic clip assembly 2 to freely rotate for a suitable angle.

In step 3, the inner guide assembly 3 is pulled backward to drive the hemostatic clip assembly 2 to move backward. The front end surface 1113 of the reduced-diameter portion 111 blocks the back end surface of the first stop ring 2311 of the hemostatic clip sleeve body 231, thereby closing the two hemostatic jaws 21 for a pre-locking action. Attention should be paid to control the force of pulling the inner guide assembly 3 backward. The inner guide assembly 3 is not pulled backward to a locked state unless the locking position is determined. The two hemostatic jaws 21 can be opened and closed freely.

In step 4, when the two hemostatic jaws 21 are in a closed but non-locked state, the inner guide assembly 3 is pushed forward. The inner conical surface 1112 of the reduced-diameter portion 111 blocks the guide surface of the second stop ring 2312, and the two hemostatic jaws 21 are opened to a maximum angle. The force of pushing the inner guide assembly 3 is controlled, so the second stop ring 2312 of the protruding portion is not pushed out of the outer tube head 11. At this time, the inner guide assembly 3 can be freely rotated to drive the frontmost hemostatic clip assembly 2 freely rotate for a suitable angle.

The step 3 and the step 4 are repeated to achieve repeated opening and closing and free rotation of the hemostatic clip assembly 2. At this time, the pin shaft 42 of the hemostatic clip assembly 2 moves back and forth within the free segments 412 of the opening/closing slots 41 of the hemostatic jaw tail plates 212.

In step 5, the step 3 is repeated.

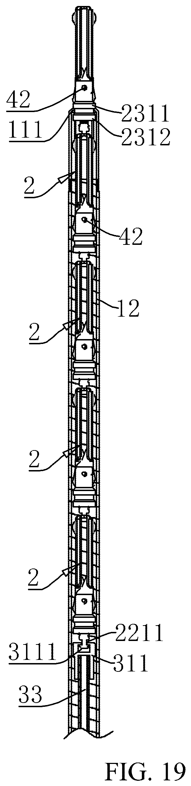

As shown in FIG. 19, in step 6, when the positions of the hemostatic jaws 21 are determined, the inner guide assembly 3 is continuously pulled backward. The hemostatic clip transmission member 22 is moved backward, and the pin shaft 42 of the opening/locking mechanism 4 enters the locking segments 411 of the opening/closing slots 41. Thus, the two hemostatic jaws 21 are locked, thereby performing a clamping and locking operation on the soft tissue.

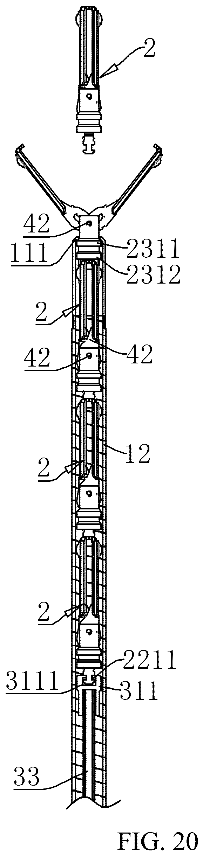

As shown in FIG. 20, in step 7, the inner guide assembly 3 is pushed forward to drive the hemostatic clip assembly 2 forward. The inner conical surface 1112 of the reduced-diameter portion 111 blocks the front side of the guide surface of the second stop ring 2312 of the protruding portion. At this time, the force required to push the second stop ring 2312 out of the outer tube head 11 is significantly less than the thrust required to unlock the two hemostatic jaws 21 clamping the tissue. The inner guide assembly 3 is continuously pushed forward to push the second stop ring 2312 of the protruding portion out of the outer tube head 11. The back hemostatic clip assembly 2 is slowly pushed out from the outer tube head 11. When the inner conical surface 1112 of the reduced-diameter portion 111 on the outer tube head 11 abuts against the guide surface of the first stop ring 2311 in the back hemostatic clip assembly 2, the two hemostatic jaws 21 are opened, releasing the previous hemostatic clip assembly 2.

The above steps are repeated for operating the subsequent hemostatic clip assemblies 2.

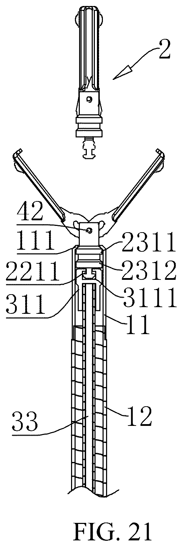

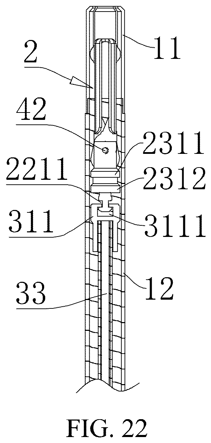

As shown in FIG. 21 and FIG. 22, after the backmost hemostatic clip assembly 2 releases the hemostatic clip assembly 2 before it, the backmost hemostatic clip assembly 2 does not perform tissue clamping. The inner guide assembly 3 is pulled back to retract the backmost hemostatic clip assembly 2 into the outer tube head 11, and is withdrawn along with a remaining component from the endoscope.

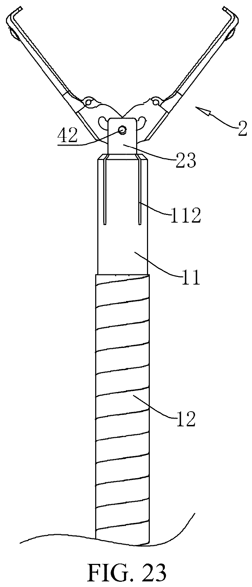

As shown in FIG. 23 to FIG. 31, the hemostatic clip assembly in Embodiment 2 is pulled as follows:
As shown in FIG. 23, in step 1, the inner guide assembly 3 is pushed forward to slowly push the hemostatic clip assembly 2 out of the outer tube head 11. The first stop ring 2311 of the hemostatic clip sleeve 23 is blocked by the inner conical surface 1112 of the reduced-diameter portion 111 of the outer tube head 11. The two hemostatic jaws 21 of the hemostatic clip assembly 2 are opened.

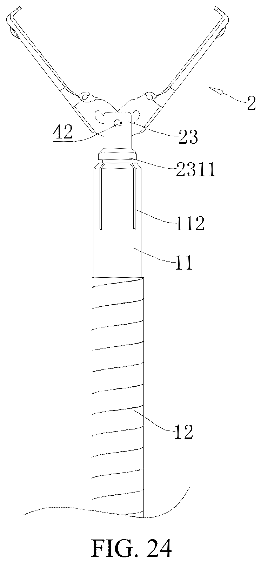

As shown in FIG. 24, in step 2, due to the notches 112 of the front end of the outer tube head 11, the front portion of the outer tube head 11 is expanded outward. The inner guide assembly 3 is continuously pushed forward to push the first stop ring 2311 of the hemostatic clip sleeve 23 out to the front end of the front end surface 1113 of the outer tube head 11. At this time, the inner guide assembly 3 can be freely rotated to drive the frontmost hemostatic clip assembly 2 to freely rotate for a suitable position for clamping the lesion tissue.

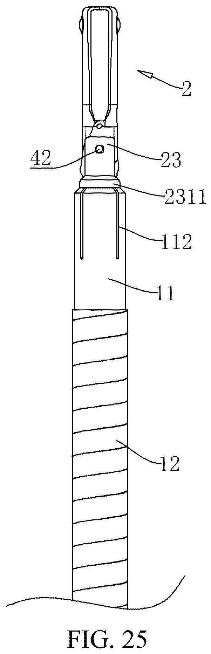

As shown in FIG. 25, in step 3, the inner guide assembly 3 is pulled backward to drive the frontmost hemostatic clip assembly 2 to move backward. The front end surface 1113 of the outer tube head 11 blocks the back end of the first stop ring 2311 of the hemostatic clip sleeve 23. The two hemostatic jaws 21 are pulled into a locked state. At this time, the frontmost hemostatic clip assembly 2 completes the action of clamping the lesion tissue. The force of pulling the inner guide assembly 3 backward is controlled, and the two hemostatic jaws are not pulled into the locked state if the clamping position is not determined.

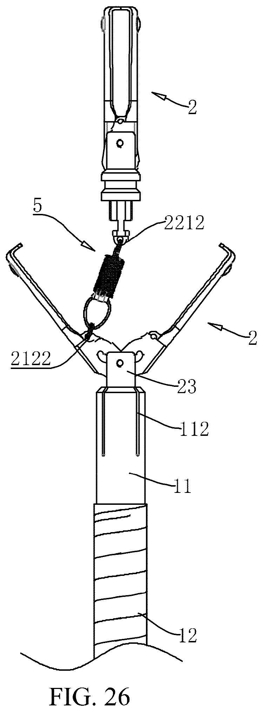

As shown in FIG. 26, in step 4, the force required to push the second stop ring 2312 of the hemostatic clip sleeve 23 out of the outer tube head 11 is significantly less than the thrust required to unlock the two hemostatic jaws 21 locked for clamping the tissue. The inner guide assembly 3 is pushed forward to drive the front hemostatic clip assembly 2 forward. Thus, the second stop ring 2312 of the hemostatic clip sleeve 23 in the front hemostatic clip assembly 2 is pushed out of the outer tube head 11, until the first stop ring 2311 of the hemostatic clip sleeve 23 in the back hemostatic clip assembly 2 is blocked by the inner conical surface 1112 of the reduced-diameter portion 111 of the outer tube head 11. Thus, the two hemostatic jaws 21 in the back hemostatic clip assembly 2 are opened, releasing the front hemostatic clip assembly 2.

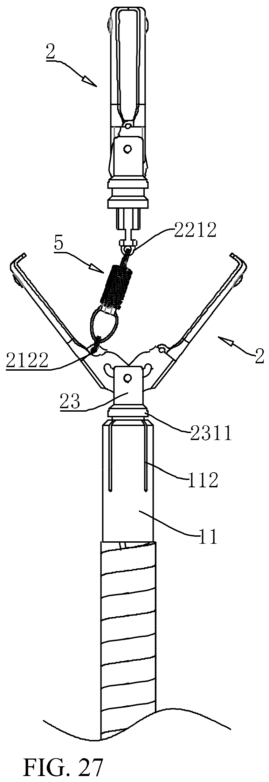

As shown in FIG. 27, in step 5, the inner guide assembly 3 is pushed forward to push the first stop ring 2311 of the hemostatic clip sleeve 23 in the back hemostatic clip assembly 2 out to the front portion of the front end surface 1113 of the outer tube head 11.

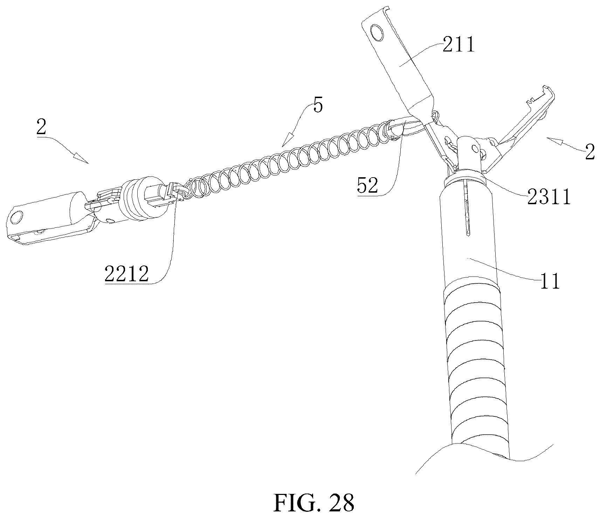

As shown in FIG. 28, in step 6, the lesion tissue is pulled. The back hemostatic clip assembly 2 is moved to an opposite side of the lesion tissue, and the clamping position on the normal tissue is determined.

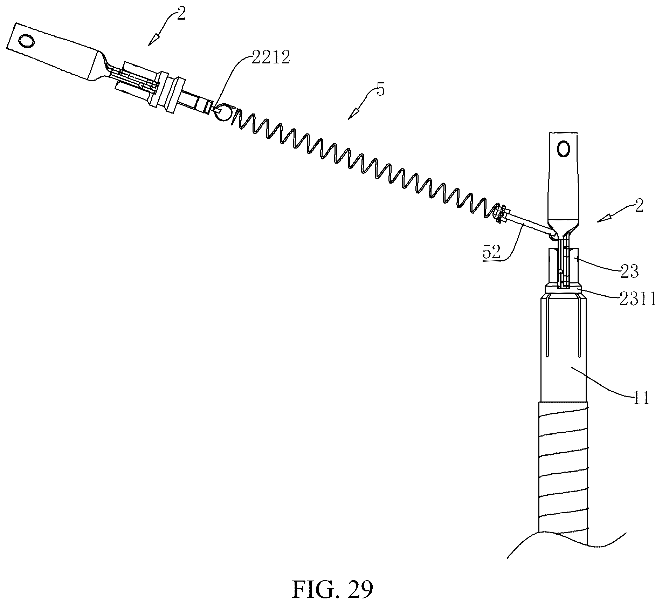

As shown in FIG. 29, in step 7, the inner guide assembly 3 is pulled backward to drive the back hemostatic clip assembly 2 to move backward within the outer tube assembly 1. The front end surface 1113 of the outer tube head 11 blocks the back end of the first stop ring 2311 of the hemostatic clip sleeve 23 in the back hemostatic clip assembly 2. The two hemostatic jaws 21 of the back hemostatic clip assembly 2 are pulled into a locked state. The back hemostatic clip assembly 2 completes the action of clamping a normal tissue, and via the elastic assembly 5, the back hemostatic clip assembly 2 pulls and lifts the lesion tissue clamped by the front hemostatic clip assembly 2 to form a high bulge.

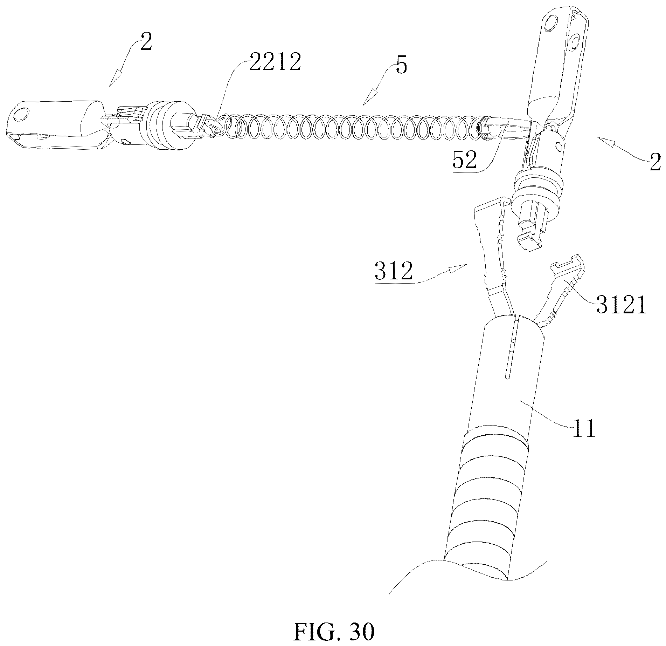

As shown in FIG. 30, in step 8, the inner guide assembly 3 is pulled forward to drive the back hemostatic clip assembly 2 forward. The second stop ring 2312 of the hemostatic clip sleeve 23 in the back hemostatic clip assembly 2 is pushed out of the outer tube head 11. The inner guide assembly 3 is continuously pushed forward. The sleeve ring 313 of the inner guide assembly 3 is blocked inside the outer tube head 11 by the inner conical surface 1112 of the reduced-diameter portion 111 of the outer tube head 11. The release clip bodies 3121 are pushed out of the outer tube head 11 and are opened, releasing the back hemostatic clip assembly 2.

In step 9, the inner guide assembly 3 is pulled backward to retract the release clip bodies 3121 back into the outer tube head 11.

At this point, all operations for the traction of the entire set of hemostatic clip assemblies 2 are completed, and the remaining component is withdrawn from the endoscope.

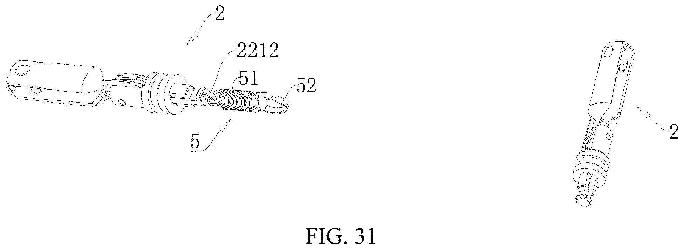

As shown in FIG. 31, after the lesion tissue is resected, the elastic ring 52 of the elastic assembly 5 is cut to disconnect the connection between the elastic ring 52 and the spring ring 53. The resected lesion tissue together with the hemostatic clip assembly 2 clamping the front side of the lesion tissue, the tension spring 51, and the elastic ring 52 are removed out of the human body. The hemostatic clip assembly 2 clamping the back side of the normal tissue and the spring ring 53 remain in the human body, waiting to be detached normally and excreted.

The operation process of Embodiment 3 is the same as that of Embodiment 1 and will not be repeated herein.

The above specific embodiments all are preferred embodiments of the present disclosure, but the protection scope of the present disclosure is not limited thereto. Therefore, all equivalent changes made in accordance with the structure, shape, and principle of the present disclosure shall fall within the protection scope of the present disclosure.

## Claims

1. A flexible, rotatable, and reopenable traction-style continuous firing hemostatic clip, comprising: an outer tube assembly (1) and multiple hemostatic clip assemblies (2) disposed within the outer tube assembly (1), wherein the multiple hemostatic clip assemblies (2) are connected sequentially end to end; the outer tube assembly (1) is further provided therein with an inner guide assembly (3) connected to a backmost hemostatic clip assembly (2); the hemostatic clip assemblies (2) each comprise two symmetrically arranged hemostatic jaws (21), a hemostatic clip transmission member (22), and a hemostatic clip sleeve (23); the hemostatic jaws (21) each comprise a front end provided with a buckle portion and a back end hinged to a front side of the hemostatic clip transmission member (22); a back side of the hemostatic clip transmission member (22) is provided with an engagement portion; the hemostatic clip transmission member (22) passes through the hemostatic clip sleeve (23), and the engagement portion extends beyond a back end of the hemostatic clip sleeve; the engagement portion is engaged with the buckle portion of an adjacent back hemostatic clip assembly (2); an opening/locking mechanism (4) for opening/closing and locking the two hemostatic jaws (21) is disposed between the two hemostatic jaws (21) and the hemostatic clip sleeve (23); an outer side of the hemostatic clip sleeve (23) is provided with a protruding portion; a front end of the outer tube assembly (1) is provided with a reduced-diameter portion (111) for limiting the hemostatic clip sleeve (23) to enable opening/closing, locking, and rotation actions of the hemostatic clip assembly (2); and the reduced-diameter portion (111) is adapted to the protruding portion.

2. The flexible, rotatable, and reopenable traction-style continuous firing hemostatic clip according to claim 1, wherein the hemostatic jaws (21) each comprise a hemostatic jaw plate (211) and a hemostatic jaw tail plate (212) fixedly connected to the hemostatic jaw plate (211); a front end of the hemostatic jaw plate (211) is fixedly connected to a hemostatic jaw element (213); the buckle portion comprises an engagement opening (2131) disposed in a middle portion of the hemostatic jaw element (213); the hemostatic jaw element (213) is provided with clip teeth (2132) located on two sides of the engagement opening (2131), respectively; and the clip teeth (2132) on the two symmetrically arranged hemostatic jaws (21) mesh with each other.

3. The flexible, rotatable, and reopenable traction-style continuous firing hemostatic clip according to claim 2, wherein the hemostatic clip transmission member (22) comprises a hemostatic clip transmission member body (221) and a hinge portion (222) fixedly connected to a front end of the hemostatic clip transmission member body (221); the engagement portion is an engagement groove (2211) disposed on a back end of the hemostatic clip transmission member body (221); the hemostatic jaw tail plate (212) is provided with a through-hole (2121) close to an end side; and the two hemostatic jaw tail plates (212) are rotatably connected to the hinge portion (222).

4. The flexible, rotatable, and reopenable traction-style continuous firing hemostatic clip according to claim 3, wherein the hinge portion (222) comprises two hemostatic jaw connecting plates (2221) fixedly connected in parallel to the front end of the hemostatic clip transmission member body (221); the two hemostatic jaw tail plates (212) are stacked between the two hemostatic jaw connecting plates (2221); a first hinge shaft (2222) passes through the two hemostatic jaw connecting plates (2221); and the first hinge shaft (2222) sequentially passes through the through-holes (2121) of the two hemostatic jaw tail plates (212).

5. The flexible, rotatable, and reopenable traction-style continuous firing hemostatic clip according to claim 3, wherein the hinge portion (222) comprises a mounting element (2224) fixedly connected to the front end of the hemostatic clip transmission member body (221); two sides of the mounting element (2224) are vertically and fixedly connected to second hinge shafts (2225), respectively; and the two second hinge shafts (2225) are embedded in the through-holes (2121) of the corresponding hemostatic jaw tail plates (212), respectively.

6. The flexible, rotatable, and reopenable traction-style continuous firing hemostatic clip according to claim 2, wherein the hemostatic clip sleeve (23) comprises a hemostatic clip sleeve body (231); the protruding portion comprises a first stop ring (2311) and a second stop ring (2312) disposed on a back end of the hemostatic clip sleeve body (231); the second stop ring (2312) is close to a back end of the hemostatic clip sleeve body (231); front sides of the first stop ring (2311) and the second stop ring (2312) are provided with inclined guide surfaces, respectively; and a front portion of the hemostatic clip sleeve body (231) is axially provided with a pair of clearance grooves (2313) for the hemostatic jaw tail plates (212) to pass through.

7. The flexible, rotatable, and reopenable traction-style continuous firing hemostatic clip according to claim 6, wherein an outer diameter of the first stop ring (2311) is adapted to an inner diameter of the outer tube assembly (1); and the outer diameter of the first stop ring (2311) is not less than an outer diameter of the second stop ring (2312).

8. The flexible, rotatable, and reopenable traction-style continuous firing hemostatic clip according to claim 3, wherein the opening/locking mechanism (4) comprises opening/closing slots (41) located in the hemostatic jaw tail plates (212), respectively; the opening/closing slots (41) are located on front sides of the through-holes (2121), respectively; the opening/closing slots (41) are L-shaped; the opening/closing slots (41) each comprise a locking segment (411) and a free segment (412) that transition smoothly; the locking segment (411) is close to a side of the hemostatic jaw plate (211); the hemostatic clip transmission member (22) is embedded in the hemostatic clip sleeve (23); a pin shaft (42) passes through a front side of the hemostatic clip sleeve body (231); and the pin shaft (42) sequentially passes through the opening/closing slots (41) of the two hemostatic jaw tail plates (212).

9. The flexible, rotatable, and reopenable traction-style continuous firing hemostatic clip according to claim 1, wherein the outer tube assembly (1) comprises an outer tube head (11) and a first flexible tube (12); the reduced-diameter portion (111) is located at a front end of the outer tube head (11); the reduced-diameter portion (111) is a reduced-diameter conical ring extending forward; the reduced-diameter portion (111) comprises an outer conical surface (1111) and an inner conical surface (1112); front ends of the outer conical surface (1111) and the inner conical surface (1112) form an annular front end surface (1113); the reduced-diameter portion (111) is circumferentially provided with multiple strip-shaped notches (112); the notches (112) extend backward to the outer tube head (11); an inner side of a back portion of the outer tube head (11) is provided with an inner boss (113); and a front end of the first flexible tube (12) is provided with an outer step (121) tightly fitted to the inner boss (113).

10. The flexible, rotatable, and reopenable traction-style continuous firing hemostatic clip according to claim 9, wherein an inner side of a back end of the first flexible tube (12) is tightly fitted and connected to a second flexible tube (13).

11. The flexible, rotatable, and reopenable traction-style continuous firing hemostatic clip according to claim 3, wherein the inner guide assembly (3) comprises a coupling portion (31), a tube sleeve (32), and an inner guidewire (33); a front end of the coupling portion (31) is engaged with the engagement groove (2211) of the backmost hemostatic clip assembly (2); the tube sleeve (32) is connected to a back end of the coupling portion (31); and the inner guidewire (33) is embedded and fixedly connected within the tube sleeve (32).

12. The flexible, rotatable, and reopenable traction-style continuous firing hemostatic clip according to claim 11, wherein the coupling portion (31) is an inner guide head (311); a front end of the inner guide head (311) is provided with a guide groove (3111) engaged with the engagement groove (2211) of the backmost hemostatic clip assembly (2); a back end of the inner guide head (311) is provided with a mounting hole (3112); the tube sleeve (32) is a flexible inner guide tube (321); the inner guidewire (33) is embedded in the flexible inner guide tube (321); and a front end of the flexible inner guide tube (321) and a front end of the inner guidewire (33) are tightly fitted and embedded in the mounting hole (3112).

13. The flexible, rotatable, and reopenable traction-style continuous firing hemostatic clip according to claim 11, wherein the coupling portion (31) comprises a release clip (312) and a sleeve ring (313); the release clip (312) comprises two symmetrically arranged release clip bodies (3121); back portions of the two release clip bodies (3121) are fixedly connected to a clip back (3124); front ends of the two release clip bodies (3121) are provided with opposite clip grooves (3122); the clip grooves (3122) are engaged with the engagement groove (2211) of the backmost hemostatic clip assembly (2); middle portions of the two release clip bodies (3121) are provided with two opposite clamping grooves (3123); the two clamping grooves (3123) are adapted to the sleeve ring (313); an outer diameter of the sleeve ring (313) is adapted to an inner diameter of the outer tube assembly (1); the tube sleeve (32) is a connecting tube (322); the clip back (3124) is fixedly connected to one end of the connecting tube (322); and another end of the connecting tube (322) is fixedly connected to the inner guidewire (33).

14. The flexible, rotatable, and reopenable traction-style continuous firing hemostatic clip according to claim 1, wherein an elastic assembly (5) is hooked between two adjacent ones of the hemostatic clip assemblies (2); the elastic assembly (5) comprises a tension spring (51), an elastic ring (52), and a spring ring (53); one end of the elastic ring (52) is fixedly connected to a connecting post (521); the connecting post (521) is provided with a shaft hole (5211); the connecting post (521) is embedded in an inner cavity of the tension spring (51), and a spring shaft (511) at one end of the tension spring (51) is embedded in the shaft hole (5211); an inner side of a middle portion of each of the hemostatic jaws (21) is provided with a traction hole (2122); a back end of the engagement portion is fixedly connected to a traction ring (2212); a spring end ring (512) at another end of the tension spring (51) is hooked to the traction ring (2212) of a front hemostatic clip assembly (2); and the spring ring (53) is hooked between the elastic ring (52) and the traction hole (2122) of a back hemostatic clip assembly (2).

15. The flexible, rotatable, and reopenable traction-style continuous firing hemostatic clip according to claim 1, wherein a front portion of each of the hemostatic jaws (21) is provided with a fluorescent portion (6); the fluorescent portion (6) is a strip-shaped piece made of a fluorescent material; the front portion of each of the hemostatic jaws (21) is further provided with a through adhesive-filling hole (2111); the fluorescent portion (6) is attached to an outer wall of the hemostatic jaw (21) and is fixedly connected to the hemostatic jaw (21) through the adhesive-filling hole (2111); and a front end of the fluorescent portion (6) extends beyond the hemostatic jaw (21) to form a pressing portion (61).
